# EUROPEAN PATENT SPECIFICATION

(11) **EP 3 013 273 B1**
(45) Date of publication and mention of the grant of the patent: **20.11.2019**
(21) Application number: 14818283.5
(22) Date of filing: 16.06.2014
(51) Int. Cl.: A61C 1/08, A61C 11/00, A61C 13/00, G06F 17/50, A61C 8/00, A61C 7/00

(54) **A METHOD AND SYSTEM FOR THE MANUFACTURING OF AN ORAL TEMPLATE FROM A 3D DIGITAL DATA**
VERFAHREN UND SYSTEM ZUR HERSTELLUNG EINER ORALEN VORLAGE AUS DIGITALEN 3D-DATEN
PROCÉDÉ ET SYSTÈME DE FABRICATION D'UN MODÈLE BUCCAL À PARTIR DE DONNÉES NUMÉRIQUES TRIDIMENSIONNELLES (3D)

(30) Priority: 27.06.2013 IL 22725813
(43) Date of publication of application: 04.05.2016
(73) Proprietor: Samrano, Sergio, 3600500 Moshav Alonei Abba (IL); Shalev, Michal, 3600500 Moshav Alonei Abba (IL)
(72) Inventor: SAMRANO, Sergio, P.O. Box 295 (IL)
(74) Representative: Boult Wade Tennant LLP
(86) International application number: PCT/IL2014/050543
(87) International publication number: WO 2014/207739

(56) References cited:
- WO-A1-2014/154584
- WO-A2-2011/101447
- US-A1- 2007 154 866
- US-A1- 2012 143 364

## Description

### Field of the Invention

The present invention relates to the field of oral templates. More particularly, the invention relates to a procedure for the production of a template for oral and dental treatments such as endodontics, total and partial restoration, prosthodontics, periodontology, orthodontics, implant prosthodontics and the like.

### Background of the invention

One aspect of oral treatment involves the use of oral templates. The treatment for the tooth must be done according to predefined medical practice parameters in order to guarantee a proper fit in terms of biological, functional and cosmetic parameters. Because of the limited space, working within a patient's mouth poses many impediments to easily and precisely providing the oral treatment, and require high skills for ultimately giving an optimal result.

In preparing a tooth for such oral treatments, however, the most commonly used method is simply to survey the area visually and drill according to the visual inspection. In other words, a dentist drills the tooth at a visually determined angle with all the constraints.

To address this problem and to protect patient from being treated incorrectly, an enhanced aiding tools for accurate oral treatment are required.

It is an object of the present invention to provide an oral template which is capable of correctly guiding a dentist while treating a tooth within the oral cavity of a patient, for endodontic treatment.

It is another object of the present invention to provide a method which is capable of providing advantage in diagnosis, dental planning, treatment and oral rehabilitation.

Other objects and advantages of the invention will become apparent as the description proceeds.

Patent application WO 2011/101447 describes designing a surgical template to guide endodontic instruments to localize the root canal(s) intra-operatively, where the guide is manufactured by means of a computer driven system (e.g. milling, rapid prototyping, etc.).

Patent application WO 2014/154584 describes a guiding template is designed in the dimensions thereof as a counterpart to the prepared cavity, wherein at least one guiding opening is arranged within the guiding template in such a way that the guiding opening points toward an entry point and direction of the root canal.

### Summary of the Invention

The present invention is defined by the appended independent claim 1. Preferred embodiments of the invention are defined by the dependent claims.

According to an embodiment of the invention the guiding paths indicate the optimal drilling depth and the optimal drilling angle required for each specific treatment.

According to an embodiment of the invention the dental template is manufactured in such a manner that a minimal drilling will be applied to the tooth.

According to an embodiment of the invention the dental template is manufactured in such a manner that it will limit the possible drilling depth of a dental tool, thereby reducing the possibilities of damaging the surroundings tissues.

According to an embodiment of the invention the recording includes a dental CT image of the at least one tooth. According to an embodiment of the invention the dental template is adapted for applying an endodontics treatment to at least one tooth.

According to an embodiment of the invention the dental template is adapted to treat a plurality of tooth at the same time.

In another aspect (not claimed), the present disclosure relates to a method for the manufacturing of dental template for optimizing dental treatment, comprising:
a. prior to a dental treatment, recording and digitizing a first three-dimensional (3D) data that represents the original anatomical structure of the surface of a missing tooth within the oral cavity of a patient, for generating a first virtual model that reflects the original anatomical structure of said surface (i.e., the virtual model of the original surface);
b. providing data representing the form of at least one tooth to be restored;
c. processing said first 3D data for generating a second virtual model that reflects the form of said surface that includes the said at least one restored tooth (i.e., the virtual model of an optimal restored missing tooth), wherein said second virtual model generated according to anatomical and functional parameters as defined by medical practice; and
d. manufacturing said optimal restored missing tooth according to said second virtual model.

According to an embodiment the recording includes a dental CT image for providing data in order to further apply a surgical drilling to the bone.

According to an embodiment the processing of the second virtual model includes at least one guiding path for applying the surgical drilling to the bone (e.g., such that a dental implant can be applied).

### Brief Description of the Drawings

In the drawings:
- Fig. 1A schematically illustrates a cross-sectional view of a digital form of a tooth as recorded and digitized from the original anatomical structure of a real tooth within the oral cavity of a patient, wherein the digital form of the tooth is marked with an ideal reshape line (not claimed);
- Fig. 1B schematically illustrates a perspective view of a dental template on top of a tooth, according to a non-claimed embodiment of the invention;
- Fig. 1C schematically illustrates a cross-sectional view of the tooth of Fig. 1A provided with a dental template on top;
- Fig. 1D schematically illustrates a cross-sectional view of the tooth of Fig. 1A as it should look-like after applying a reshaping treatment with the aid of the dental template of Fig. 1B;
- Fig. 1E schematically illustrates a digital form of the portion that need to be extracted from the original tooth;
- Fig. 2A schematically illustrates a cross-sectional view of a digital form of a tooth as recorded and digitized from the original anatomical structure of a real tooth within the oral cavity of a patient, and provided with an endodontics dental template on top, according to an embodiment of the invention;
- Fig. 2B schematically illustrates a perspective view of the endodontics dental template, according to an embodiment of the invention;
- Fig. 2C schematically illustrates a perspective view of the tooth of Fig. 2A after applying a drilling treatment with the aid of the endodontics dental template of Fig. 2B;
- Fig. 2D schematically illustrates a digital form of the portion that need to be extracted from the original tooth with the aid of the endodontics dental template;
- Fig. 2E schematically illustrates a cross-sectional view of the upper portion of Fig. 2C;
- Fig. 2F schematically illustrates a perspective view of the endodontics dental provided with driller guiding tubes, according to an embodiment of the invention;
- Fig. 2G schematically illustrates a cross-sectional view of a tooth which demonstrates the position the guiding tubes with respect to the root canals of the tooth, according to an embodiment of the invention;
- Figs. 3A-3G show a dental template for applying a minimal treatment required for preparing a tooth for the restoration, according to a non-claimed embodiment;
- Figs. 4A-4E schematically illustrate a periodontal template and its application to the gingiva, according to a non-claimed embodiment;
- Figs. 5A-5B schematically illustrate an orthodontics template, according to a non-claimed embodiment;
- Figs. 6A-6C schematically illustrate an implant prosthodontics template for obtaining proper fit of the final implant prosthodontics, according to a non-claimed embodiment;
- Fig. 7 schematically illustrates a computing environment in which the invention may be implemented, according to an embodiment of the invention.

### Detailed Description of the Invention

Throughout this description the term "oral template" is used to indicate a template adapted to guide an oral treatment tool within the oral cavity. This term does not imply any particular shape, construction material or geometry, and invention is applicable to a variety of dental and oral treatments, such as endodontics, total and partial restoration, prosthodontics, periodontology, orthodontics, implant prosthodontics. In particular, throughout this description the term "dental template" is used to indicate a template adapted to guide a dental drilling tool while treating the tooth. This term does not imply any particular shape, construction material or geometry.

Reference will now be made to several embodiments of the present invention, examples of which are illustrated in the accompanying figures. Wherever practicable similar or like reference numbers may be used in the figures and may indicate similar or like functionality. The figures depict embodiments of the present invention for purposes of illustration only. One skilled in the art will readily recognize from the following description that alternative embodiments of the structures and methods illustrated herein may be employed without departing from the principles of the invention described herein.

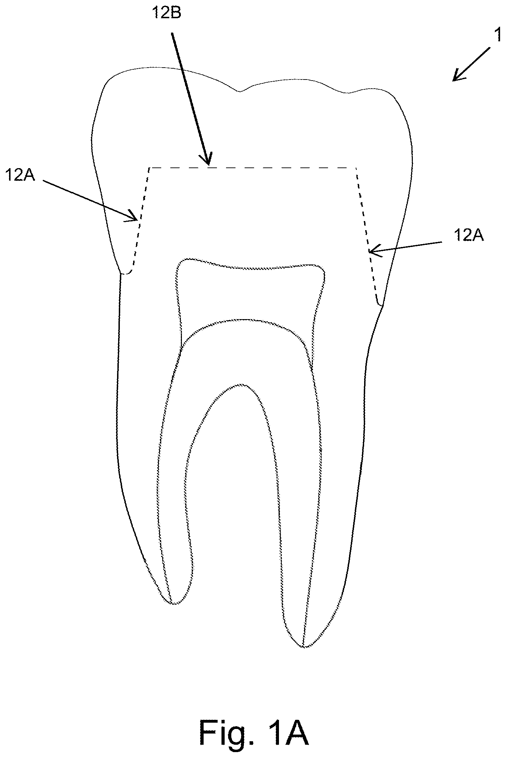

Fig. 1A schematically illustrates a cross-sectional view of a digital form of a typical tooth 1 within the oral cavity, as recorded and digitized from the original anatomical structure of a real tooth within the oral cavity of a patient. The dotted lines 12A at the upper end of tooth 1 indicate an ideal reshape line according to which an optimal drilling angle should be applied to the original real tooth in order to prepare the tooth (i.e., reshape its structure) as required for applying a specific treatment, e.g., a dental prosthesis. A dentist may further apply additional treatment to the occlusal surface of tooth 1, by essentially flattening the occlusal surface as indicated by the dotted line 12B. For example, according to contemporary medical practice the optimal drilling angle to prepare a tooth for a dental prosthesis should be about 9 degrees (perpendicular to the dotted line 12B).

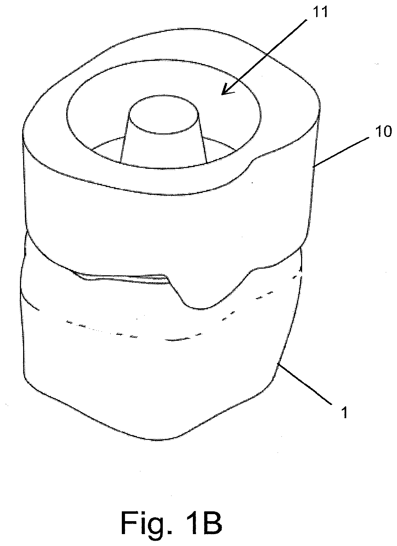

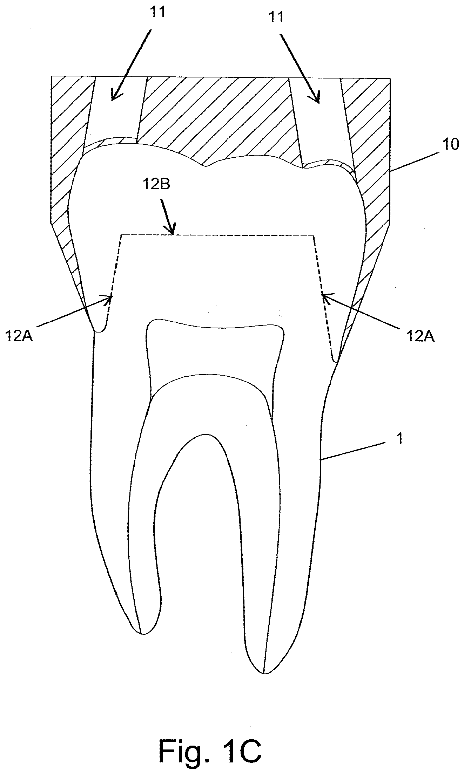

Fig. 1B shows a dental template (positioned on top of tooth 1) that can be used in conjunction with methods disclosed herein. The dental template illustrated in this figure is particularly convenient because it can be applied to original tooth before carrying out any alterations in the structure of the tooth. The dental template generally indicated by numeral 10 in the figure comprises a guiding path(s) 11 for a dental drilling tool in order to obtain the optimal drilling angle as indicated by the dotted lines 12A of Fig. 1A. Fig. 1C schematically illustrates a cross-sectional view of tooth 1 provided with the dental template 10 on top.

The process of manufacturing such dental templates will be explained hereinafter, with respect to several embodiments of the present invention.

Initial stage: The process of creating the dental template 10 such that it will be able to be mounted on top of a corresponding real tooth may involve the following steps:
- prior to the dental treatment, recording and digitizing a first three-dimensional (3D) data that represents the original anatomical structure of tooth 1 within the oral cavity of a patient;
- processing the first 3D data for generating a first virtual model that reflects the original anatomical structure of tooth 1 and a second virtual model that reflects the form of tooth 1 as it should be after the applying of an optimal treatment (e.g., as tooth 1 should look like after the reshaping of the real tooth at an optimal drilling angle);
- generating a third virtual model that represents the dental template 10 according to the first and second virtual models, such that the virtual model of the dental template 10 includes the guiding path 11 for an optimal dental treatment. The guiding path 11 is determined according to predefined medical practice parameters for optimal treatment. The inner surface of the dental template 10 reflects the reverse form of tooth 1, in particular the reverse form of the occlusal surface; and
- manufacturing the dental template 10 according to the third virtual model, such that a dental treatment can be applied to the real tooth with a dental tool via the guiding path 11 whenever the dental template 10 is mounted on top of the real tooth (e.g., as simulated in Fig. 1C).

According to an embodiment of the present invention, dental template 10 can be manufactured by using a process of making a three-dimensional solid object of virtually any shape from a digital model, such as additive manufacturing or 3D printing.

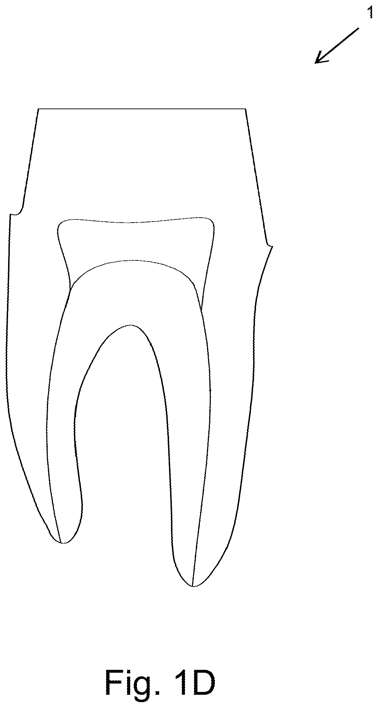

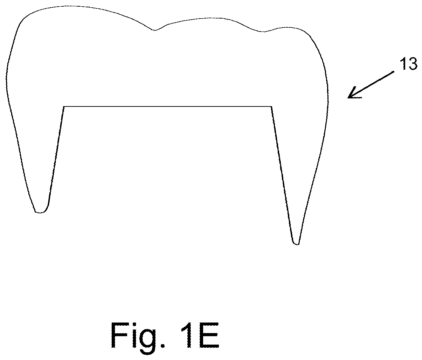

Fig. 1D schematically illustrates a cross-sectional view of the tooth 1 as it should look-like after applying a reshaping treatment with the aid of the dental template 10. This figure simulates the shaped structure of tooth 1 after applying the dental treatment to it with dental template 10. Fig. 1E schematically illustrates a digital form of a portion 13 that should be extracted from tooth 1 and accordingly from the real tooth.

According to a non-claimed embodiment, after applying the aforementioned dental treatment to the real tooth with dental template 10 (as simulated by tooth 1 in Figs. 1A-1E), one or more of the following tasks can be further performed:
- recording and digitizing a second 3D data that represents the anatomical structure of the real tooth after its reshaping with dental template 10 (hereinafter the treated tooth and that should look similar to the form of tooth 1 in Fig. 1D);
- digitally comparing the anatomical structure of the treated tooth with the second virtual model that reflects the optimal form of the treated tooth (i.e., as illustrated in Fig. 1D), in order to verify the quality of the actual treatment with respect to the expected optimal one.

Assuming that the results of the treatment to the treated tooth with dental template 10 are optimal, a dentist may further apply additional treatment to the occlusal surface of the treated tooth, if required (e.g., as indicated by the dotted line 12B in Fig. 1C.

All the above will be better understood through the following illustrative and non-limitative examples. In general, the following are examples for applying further treatments to a tooth of a patient in an optimal manner with respect to the given medical practice parameters.

### Example 1: Dental Prosthesis (not claimed)

The process of creating an essentially optimal dental prosthesis for a tooth may involve the following steps (in addition to the aforementioned initial stage):
- recording and digitizing a second 3D data that represents the anatomical structure of the treated tooth (i.e., as indicated by tooth 1 in Fig. 1D);
- generating a virtual model of a dental prosthesis according to the first and second 3D data of tooth 1 as obtained in the aforementioned initial stage, such that the restorations of the original anatomical structure could be accurately reconstructed by a dental prosthesis replacement; and
- manufacturing a dental prosthesis replacement for the real tooth according to said virtual model of said dental prosthesis (e.g., by 3D printing).

As will be appreciated by the skilled person the arrangement described in the figures results in a method that allows obtaining an essentially optimal dental treatments and optimal reshaping results to the teeth within the oral cavity of a patient. Moreover, such treatment minimizes, or may even completely eliminates, the chances of damaging the treated tooth or teeth. Furthermore, the method suggested by the present invention allows the restoration of the original anatomical structure of the tooth.

### Example 2: Endodontics

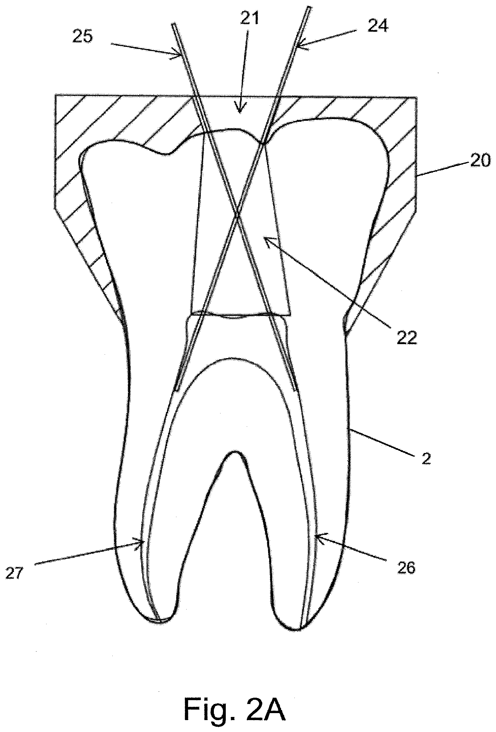

Fig. 2A schematically illustrates a cross-sectional view of a digital form of a tooth 2 as recorded and digitized from the original anatomical structure of a real tooth within the oral cavity of a patient, and provided with an endodontics dental template 20 on top. The lines 24 and 25 indicate the optimal drilling angle required for the preparation of the tooth for an endodontic treatment. In this case, the drilling should be applied such that an access path, in an appropriate angle, to the root canals 26 and 27 of the tooth pulp will be obtained.

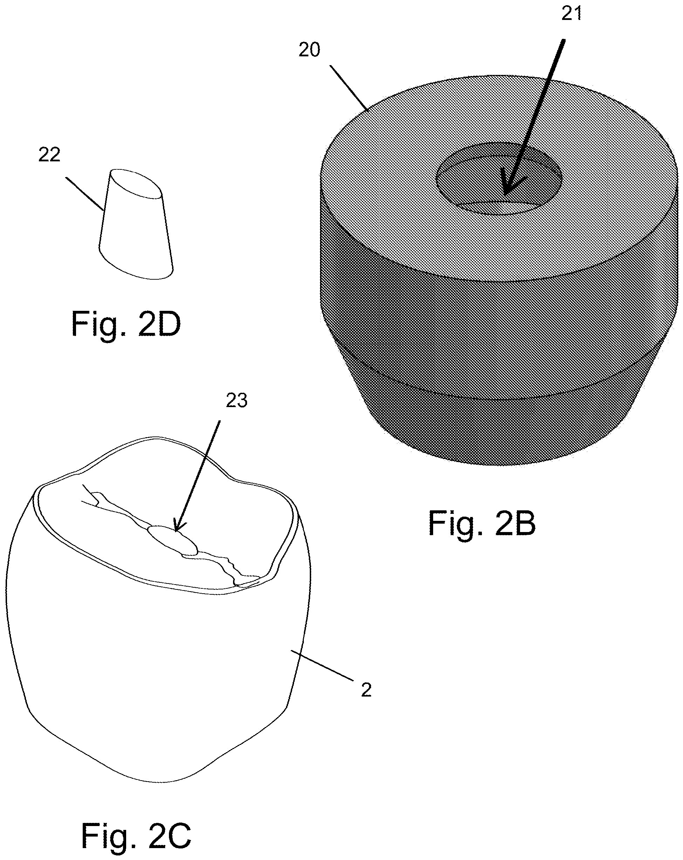

Fig. 2B schematically illustrates a perspective view of the endodontics dental template 20, according to an embodiment of the invention. The endodontics dental template 20 as illustrated in this figure is particularly convenient because it can be applied to the real tooth before carrying out any alterations in the structure of the real tooth. Dental template 20 comprises a guiding path 21 for a dental drilling tool in order to obtain the optimal drilling angle and depth as indicated by the lines 24 and 25 and the area 22 of Fig. 2A.

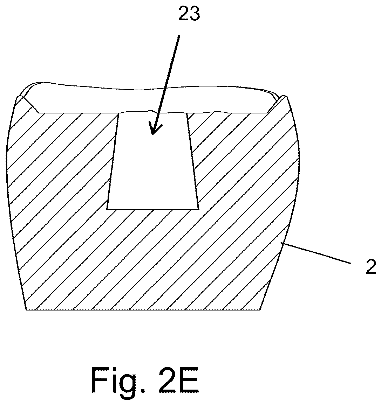

Fig. 2C schematically illustrates a perspective view of tooth 2 after applying a drilling treatment with the aid of the endodontics dental template 20. Fig. 2D schematically illustrates a digital form of a portion 22 that need to be extracted from the original real tooth with the aid of an actual endodontics dental template. Fig. 2E schematically illustrates a cross-sectional view of the upper portion tooth 2 after the drilling treatment (i.e., after portion 22 was extracted).

According to an embodiment of the invention, the procedure for the generation of the dental template 20 may further involve the use of dental Computed Tomographic (CT) imaging in addition to the recording of the 3D data as aforementioned hereinabove. The CT imaging provides the system with information from multiple panoramic and cross-sectional views of the teeth for the creation of virtual models of a corresponding template (e.g., the location of a tooth's root canal).

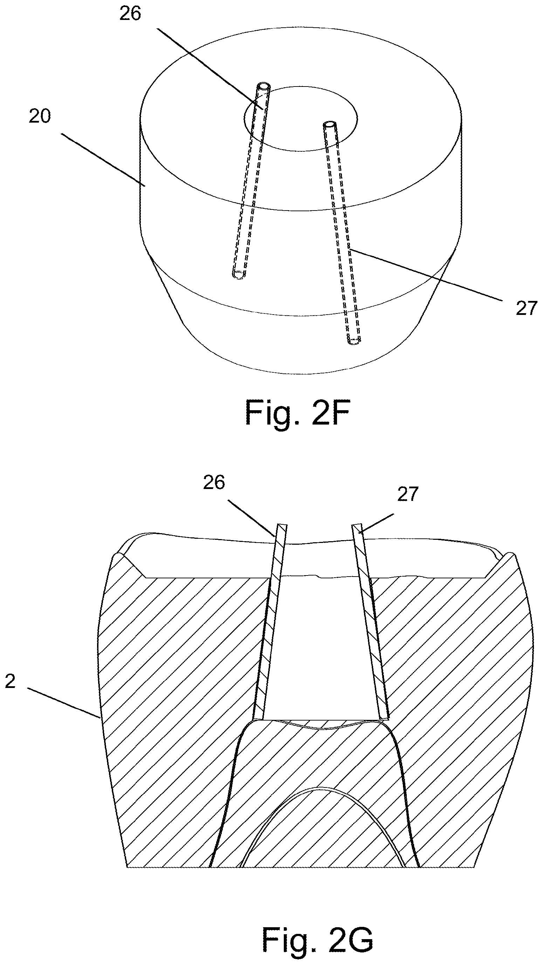

Fig. 2F schematically illustrates a perspective view of the endodontics dental 20 provided with driller guiding tubes 26 and 27, according to an embodiment of the invention. The driller guiding tubes 26 and 27 adapted to receive the driller in order to guide it directly towards the root canals, essentially in an optimal angle. Fig. 2G schematically illustrates a cross-sectional view of tooth 2 which demonstrates the position the guiding tubes 26 and 27 with respect to the root canals of tooth 2, according to an embodiment of the invention.

### Example 3: Restoration (not claimed)

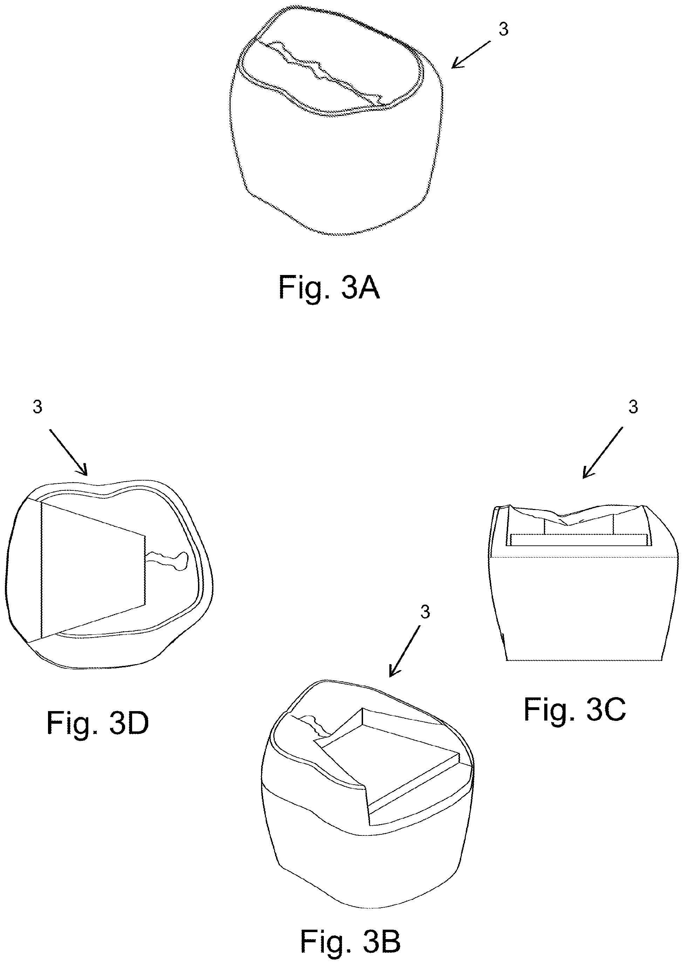

The following example and accompanying figures refers to a dental template for applying a minimal treatment required for preparing a tooth 3 for restoration, according to an embodiment of the invention. Fig. 3A schematically illustrates a perspective view of tooth 3 before the preparation treatment. Fig. 3B schematically illustrates a perspective view of tooth 3 after the preparation treatment. Fig. 3C schematically illustrates a front view of tooth 3 after the preparation treatment and Fig. 3D schematically illustrates a top view of tooth 3 after the preparation treatment.

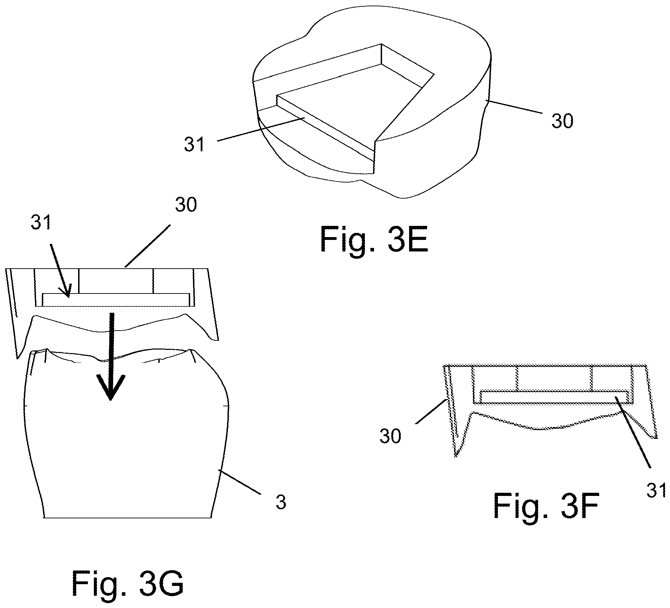

Fig. 3E schematically illustrates a perspective view of a dental template for applying a minimal treatment required for preparing tooth 3 for restoration, according to an embodiment of the invention. The dental template illustrated in this figure is particularly convenient because it can be applied to tooth 3 while allowing limiting the drilling action treatment to the minimum possible depth (i.e., the optimal drilling required). The dental template generally indicated by numeral 30 in the figure comprises a depth limiter 31 for a dental drilling tool in order to obtain the optimal drilling depth. Fig. 3F schematically illustrates a front view of dental template 30.

The depth limiter 31 sets the optimal drilling depth required for the preparation of the tooth for a partial restoration. In this example, the drilling should be applied such that the driller will remove the minimum possible layers at the damaged area of tooth 3, as shown with respect to Fig. 3B. Fig. 3G schematically illustrates placing of template 30 on top of tooth 3 before the preparation treatment.

### Example 4: Periodontology (not claimed)

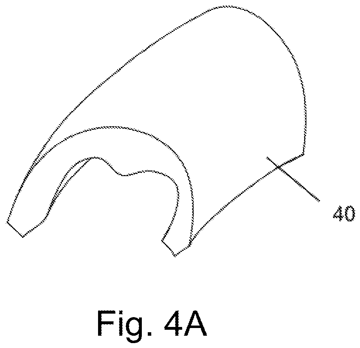

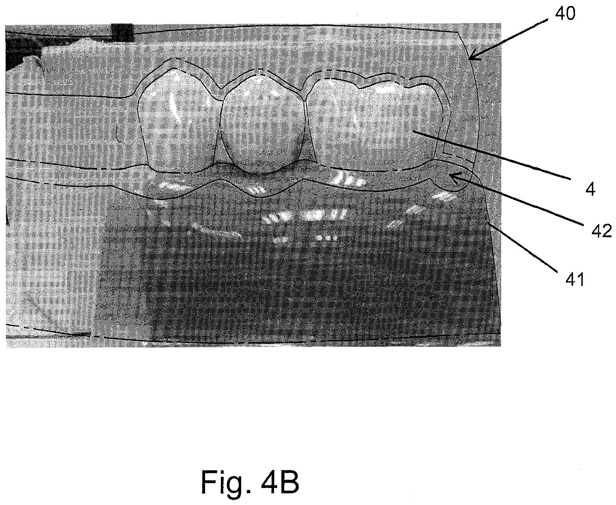

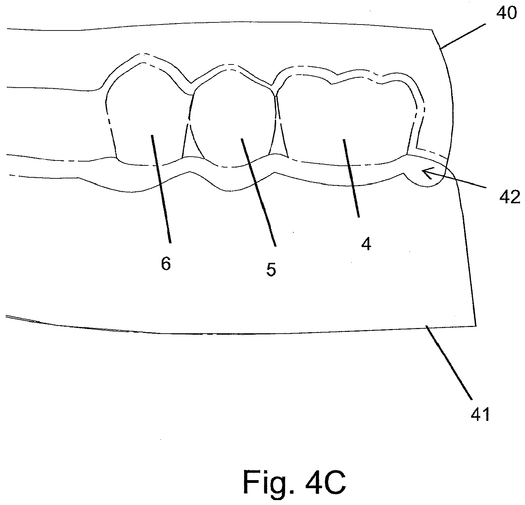

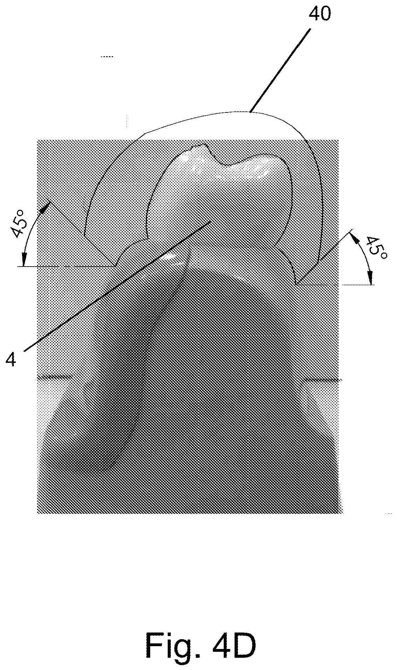

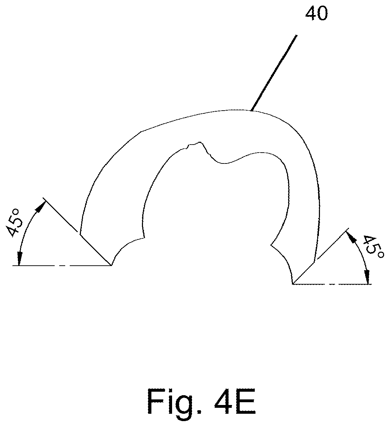

A periodontal surgery usually requires removing the non-healthy layer of the gingiva in an optimal determined angle and depth as required for each specific treatment. According to an embodiment of the invention, a procedure for manufacturing a periodontal template for obtaining optimal treatment results may involve the following steps, which are accompanied by Figs. 4A-4E (for purpose of illustration only, dental models by Nissin Dental Products INC were used in Figs. 4B and 4D to demonstrate the teeth and the gingiva):
- prior to the dental treatment, recording and digitizing a first three-dimensional (3D) data that represents the original anatomical structure of periodontal area within the oral cavity of a patient that includes the surrounding teeth.
- Measuring clinical parameters of the periodontal socket by periodontal tools and/or via radiology imaging.
- Providing the measured clinical parameters (e.g., depth of the periodontal socket) manually by a user or automatically according to the radiology imaging.
- Generating a 3D digital model of the periodontal area to be treated according to the provided clinical parameters and the recorded periodontal area (e.g., see Figs. 4B and 4C or, alternatively, Figs. 4D and 4E).
- Generating a virtual template 41 of the periodontal area according to 3D digital model. The virtual template includes an occlusal support surface 40 of the teeth at the periodontal area (as demonstrated by teeth 4-6 in Figs. 4B and 4C and by tooth 4 in Fig. 4D) and a surgical guiding path 42 along the gingiva line (Figs. 4B and 4C). The surgical guiding path 42 is determined according to the following parameters: the provided clinical parameters (e.g., socket depth), an optimal cutting incline (e.g., 45 degrees as shown in Figs. 4D and 4E) with respect to the axial perpendicular line parameter of the teeth.
- Manufacturing a periodontal template 40 according to the virtual model, such that when placing the template on top of the occlusal surface of the teeth, the guiding path 42 will face the gingiva area to be treated.

### Example 5: Orthodontics (not claimed)

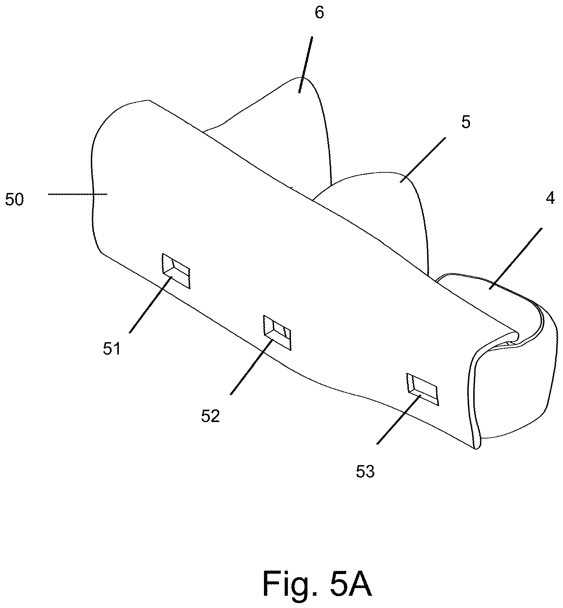

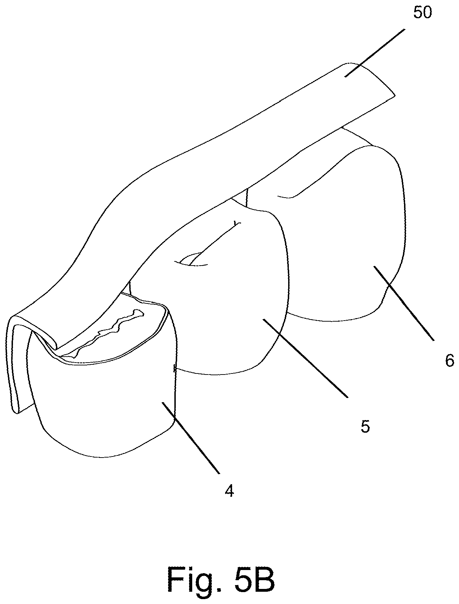

Figs. 5A-5B schematically illustrate an orthodontics template 50, according to an embodiment of the present invention.

According to an embodiment of the invention, a procedure for manufacturing an orthodontics template may involve the following steps:
- prior to the dental treatment, recording and digitizing a first three-dimensional (3D) data that represents the original anatomical structure of the teeth within the oral cavity of a patient.
- Generating a virtual model of the teeth (as indicated by numerals 4-6) that includes the location and size of each orthodontic bracket on each tooth, according to the orthodontic parameters and as defined by an orthodontic plane.
- Manufacturing the orthodontic template 50 according to the virtual model which includes openings (as indicated by numerals 51-53) according to the location and size of each orthodontic bracket, such that when placing the template on top of the incisal and buccal/palatinal surfaces, an access is provided via said openings for applying the orthodontic treatment (e.g., etching and cementing) and the corresponding brackets.

### Example 6: Implant Prosthodontics (not claimed)

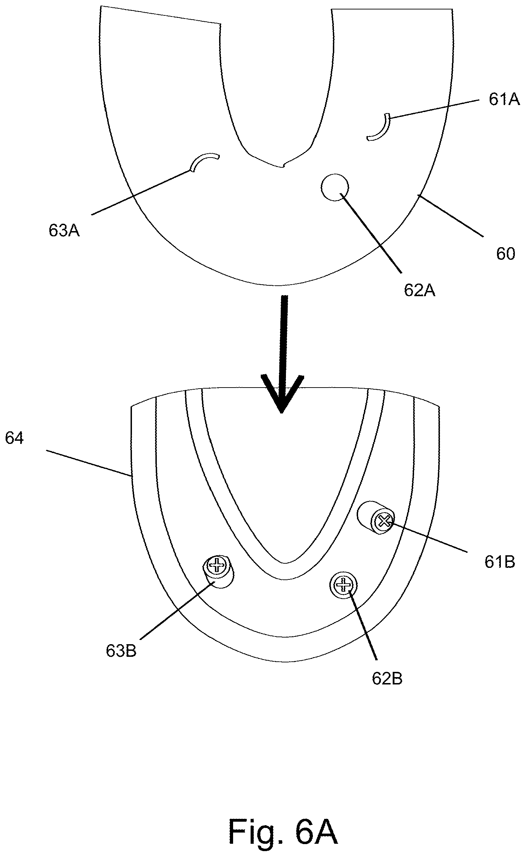

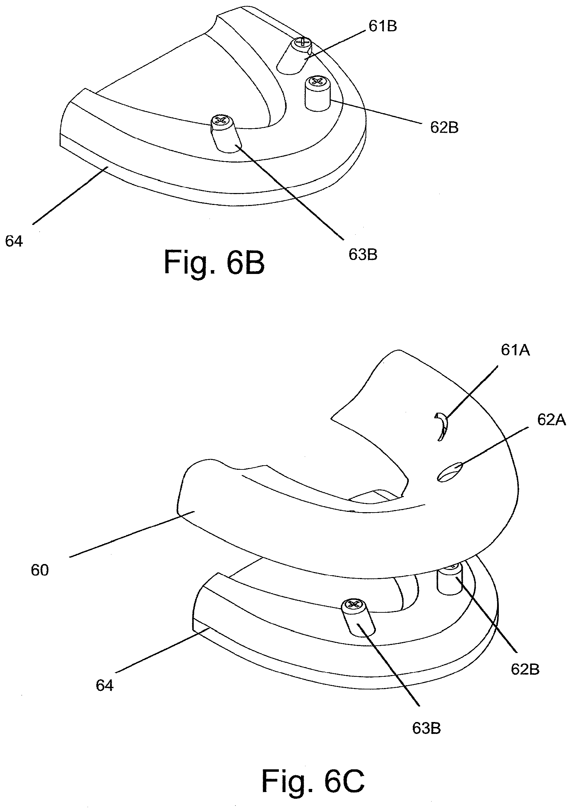

Usually the orientation of dental implants deployment in the oral cavity requires fixing the dental implant abutments, such that the final implant prosthodontics will be able to proper fit on top of them. According to an embodiment of the invention, a procedure for manufacturing an implant prosthodontics template for obtaining proper fit of the final implant prosthodontics may involve the following steps, which are accompanied by Figs. 6A-6C:
- recording and digitizing a first three-dimensional (3D) data that represents the deployment of dental implant abutments within the oral cavity of a patient (as indicated by numerals 61B-63B).
- Generating a virtual model of the dental implant abutments 61B-63B.
- Generating a virtual model of the fixed implant abutments according to the parameters that define the required insertion path of the final implant prosthodontics.
- Generating a virtual template 60 according to the virtual model of the fixed implant abutments, for allowing correcting improper orientation of the dental implant abutments, such that the final implant prosthodontics will fit the corrected dental implant abutments in an optimal manner.
- Manufacturing an implant prosthodontics (according to the virtual template 60) for allowing reshaping the form of one or more abutments such that the deployment of all the dental implant abutments together will provide the required insertion path. The reshaping may involve grinding specific areas (at abutments 61B-63B) by one or more guiding paths (61A-63A) as appear on the implant prosthodontics template.

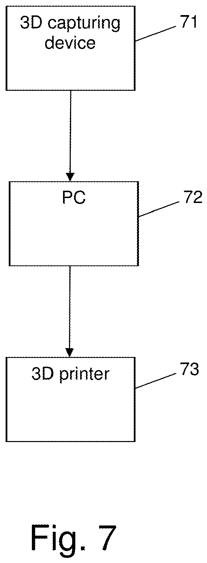

Turning now to the computer environment, Fig. 7 is intended to provide a brief, general description of a suitable computing environment in which the invention may be implemented. While the computing environment refers to general context of program modules that execute in conjunction with an application program that runs on an operating system on a personal computer 72, those skilled in the art will recognize that the invention may also be implemented in combination with other program modules. Moreover, those skilled in the art will appreciate that the invention may be practiced with other computer system configurations, including hand-held devices, multiprocessor systems, microprocessor-based or programmable consumer electronics, minicomputers, mainframe computers, and the like. The invention may also be practiced in distributed computing environments where tasks are performed by remote processing devices that are linked through a communications network. In a distributed computing environment, program modules may be located in both local and remote memory storage devices.

Embodiments of the invention for recording and digitizing the aforementioned 3D data and for creating the virtual models of the tooth and templates may partially involve a computer process (method), a computing system, or as an article of manufacture, such as a computer program product or computer readable media. The computer program product may be a computer storage media readable by a computer system and encoding a computer program of instructions for executing a computer process. For example, recording of the 3D data of one or more tooth can be obtained by using digital dentistry equipment (as indicated by numeral 71) such as CAD/CAM products by Sirona Dental Systems GmbH. The dental templates can be manufactured by using a process of making a three-dimensional solid object of virtually any shape from a digital model, such as a 3D printer 73.

Further, with respect to the example processes described herein, not all the process states need to be reached, nor do the states have to be performed in the illustrated order. The terms, "for example", "e.g.", "optionally", as used herein, are intended to be used to introduce nonlimiting examples.

All the above description and examples have been given for the purpose of illustration and are not intended to limit the invention in any way. Many different mechanisms, methods of analysis, electronic and logical elements can be employed, all without exceeding the scope of the invention.

## Claims

1. A method for the manufacturing of a dental template from a 3D digital data, comprising:
a. prior to a dental treatment, recording and digitizing a first three-dimensional (3D) data that represents the original anatomical structure of at least one tooth within the oral cavity of a patient, for generating a first virtual model that reflects the original anatomical structure of said at least one tooth;
b. processing said first 3D data for generating a second virtual model that reflects the form of said at least one tooth after applying an optimal endodontics treatment as defined by medical practice parameters;
c. generating a third virtual model of a dental template to be mounted on top of said at least one tooth according to said first and second virtual models, wherein said dental template includes a guiding path (21) for achieving an optimal drilling angle required for the preparation of the tooth for an endodontics treatment as defined by the medical practice parameters, wherein the drilling angle is such that an access path (22), in an appropriate angle, to two root canals of the tooth pulp will be obtained; and
d. manufacturing said dental template according to said third virtual model, such that a dental treatment can be applied to said at least one tooth with a dental tool via the guiding path of said dental template whenever said dental template is mounted on top of said at least one tooth, such that after finishing applying the dental treatment to the at least one tooth with the dental template an essentially optimal treated tooth is obtained.

2. A method according to claim 1, wherein the guiding path indicates the optimal drilling depth and the optimal drilling angle required for each specific treatment.

3. A method according to claim 1, wherein the dental template is manufactured in such a manner that it will limit the possible drilling depth of a dental tool, thereby reducing the possibilities of damaging surrounding tissues.

4. A method according to claim 1, wherein the recording includes a dental CT image of the at least one tooth.

5. A method according to claim 1, wherein the dental template includes at least two driller guiding tube (26, 27) adapted to receive the driller in order to guide it directly towards the two root canals.

6. A method according to claim 1, wherein the dental template is adapted to treat a plurality of teeth at the same time.

7. A method according to claim 1, wherein the guiding path comprises an orifice following the shape of a conical frustum and wherein the walls of the orifice are configured to guide a dental tool to obtain an access path, in an appropriate angle, to the at least two root canals of the tooth pulp.

8. A method according to claim 5, wherein said at least two driller guiding tubes are configured for guiding the driller towards the at least two root canals essentially in an optimal angle for each root canal.

## Patentansprüche

1. Verfahren zur Herstellung einer Zahnvorlage aus digitalen 3D-Daten, Folgendes umfassend:
a. vor einer Zahnbehandlung, Aufzeichnen und Digitalisieren erster dreidimensionaler (3D)-Daten, die die ursprüngliche anatomische Struktur von mindestens einem Zahn innerhalb der Mundhöhle eines Patienten darstellen, zum Erzeugen eines ersten virtuellen Modells, das die ursprüngliche anatomische Struktur des mindestens einen Zahnes widerspiegelt;
b. Verarbeiten dieser ersten 3D-Daten zum Erzeugen eines zweiten virtuellen Modells, das die Form des mindestens einen Zahnes, nach Anwendung einer optimalen Endodontiebehandlung, wie durch Praxisparameter definiert, widerspiegelt;
c. Erzeugen eines dritten virtuellen Modells einer Zahnvorlage, die auf dem mindestens einen Zahn gemäß dem ersten und zweiten virtuellen Modell befestigt werden soll, wobei die Zahnvorlage einen Führungsweg (21) zum Erzielen eines optimalen Bohrwinkels einschließt, der für die Präparation des Zahnes für eine Endodontiebehandlung erforderlich ist, wie durch die Praxisparameter definiert, wobei der Bohrwinkel so ist, dass ein Zugangsweg (22) in einem geeigneten Winkel zu mindestens zwei Wurzelkanälen der Zahnpulpa erhalten wird; und
d. Herstellen der Zahnvorlage nach dem dritten virtuellen Modell, so dass eine Zahnbehandlung auf den mindestens einen Zahn mit einem Dentalinstrument über den Führungsweg der Zahnvorlage angewendet werden kann, wenn die Zahnvorlage auf dem mindestens einen Zahn montiert ist,
so dass nach Beendigung der Anwendung der Zahnbehandlung auf den mindestens einen Zahn mit der Zahnvorlage ein im Wesentlichen optimal behandelter Zahn erhalten wird.

2. Verfahren nach Anspruch 1, wobei der Führungsweg die optimale Bohrtiefe und den optimalen Bohrwinkel anzeigt, die jeweils für die bestimmte Behandlung erforderlich sind.

3. Verfahren nach Anspruch 1, wobei die Zahnvorlage so hergestellt ist, dass sie die mögliche Bohrtiefe eines Dentalinstruments begrenzt und damit die Möglichkeiten der Beschädigung von umliegendem Gewebe reduziert.

4. Verfahren nach Anspruch 1, wobei die Aufzeichnung ein dentales CT-Bild des mindestens einen Zahnes einschließt.

5. Verfahren nach Anspruch 1, wobei die Zahnvorlage mindestens zwei Bohrerführungsrohre (26, 27) einschließt, die zur Aufnahme des Bohrers ausgelegt sind, um ihn direkt zu den beiden Wurzelkanälen zu führen.

6. Verfahren nach Anspruch 1, wobei die Zahnvorlage dazu ausgelegt ist, eine Mehrzahl von Zähnen gleichzeitig zu behandeln.

7. Verfahren nach Anspruch 1, wobei der Führungsweg eine Öffnung umfasst, die der Form eines konischen Stumpfes folgt, und wobei die Wände der Öffnung konfiguriert sind, um ein Dentalinstrument zu führen, um einen Zugangsweg in einem geeigneten Winkel zu den mindestens zwei Wurzelkanälen der Zahnpulpa zu erhalten.

8. Verfahren nach Anspruch 5, wobei die mindestens zwei Bohrerführungsrohre konfiguriert sind, um den Bohrer zu den mindestens zwei Wurzelkanälen im Wesentlichen in einem optimalen Winkel für jeden Wurzelkanal zu führen.

## Revendications

1. Procédé de fabrication d'un gabarit dentaire à partir de données numériques 3D, comprenant les étapes consistant à :
a. avant un traitement dentaire, enregistrer et numériser des premières données tridimensionnelles (3D) qui représentent la structure anatomique d'origine d'au moins une dent dans la cavité buccale d'un patient, pour générer un premier modèle virtuel qui reflète la structure anatomique d'origine de ladite au moins une dent ;
b. traiter lesdites premières données 3D pour générer un deuxième modèle virtuel qui reflète la forme de ladite au moins une dent après l'application d'un traitement endodontique optimal tel que défini par des paramètres de pratique médicale ;
c. générer un troisième modèle virtuel d'un gabarit dentaire à monter sur la partie supérieure de ladite au moins une dent selon lesdits premier et deuxième modèles virtuels, où ledit gabarit dentaire comporte un trajet de guidage (21) pour obtenir un angle de perforation optimal requis pour la préparation de la dent pour un traitement endodontique tel que défini par les paramètres de pratique médicale, où l'angle de perforation est tel qu'un trajet d'accès (22), selon un angle approprié, à deux canaux radiculaires de la pulpe dentaire sera obtenu ; et
d. fabriquer ledit gabarit dentaire selon ledit troisième modèle virtuel, de sorte qu'un traitement dentaire puisse être appliqué à ladite au moins une dent avec un outil dentaire par l'intermédiaire du trajet de guidage dudit gabarit dentaire chaque fois que ledit gabarit dentaire est monté sur la partie supérieure de ladite au moins une dent, de sorte que, après la fin de l'application du traitement dentaire à l'au moins une dent avec le gabarit dentaire, une dent traitée essentiellement optimale soit obtenue.

2. Procédé selon la revendication 1, dans lequel le trajet de guidage indique la profondeur de perforation optimale et l'angle de perforation optimal requis pour chaque traitement spécifique.

3. Procédé selon la revendication 1, dans lequel le gabarit dentaire est fabriqué de manière à ce qu'il limite la profondeur de perforation possible d'un outil dentaire, réduisant ainsi les possibilités d'endommager les tissus environnants.

4. Procédé selon la revendication 1, dans lequel l'enregistrement comporte une image CT dentaire de l'au moins une dent.

5. Procédé selon la revendication 1, dans lequel le gabarit dentaire comporte au moins deux tubes de guidage de fraise (26, 27) adaptés pour recevoir la fraise afin de la guider directement vers les deux canaux radiculaires.

6. Procédé selon la revendication 1, dans lequel le gabarit dentaire est adapté pour traiter une pluralité de dents en même temps.

7. Procédé selon la revendication 1, dans lequel le trajet de guidage comprend un orifice épousant la forme d'un tronc conique et dans lequel les parois de l'orifice sont configurées pour guider un outil dentaire afin d'obtenir un trajet d'accès, selon un angle approprié, aux au moins deux canaux radiculaires de la pulpe dentaire.

8. Procédé selon la revendication 5, dans lequel lesdits au moins deux tubes de guidage de fraise sont configurés pour guider la fraise vers les au moins deux canaux radiculaires essentiellement selon un angle optimal pour chaque canal radiculaire.
